# EUROPEAN PATENT SPECIFICATION

(11) **EP 2 768 508 B1**
(45) Date of publication and mention of the grant of the patent: **06.07.2016**
(21) Application number: 12784753.1
(22) Date of filing: 19.10.2012
(51) Int. Cl.: A61P 25/00, A61K 31/496, A61K 9/08, A61K 31/185, A61K 31/191, A61K 31/194, A61K 31/198, A61K 31/661, A61K 47/02, A61K 47/12, A61K 9/00

(54) **SOLUTION FOR ORAL ADMINISTRATION**
LÖSUNG ZUR ORALEN VERABREICHUNG
SOLUTION DESTINÉE À ÊTRE ADMINISTRÉE PAR VOIE ORALE

(30) Priority: 19.10.2011 US 201161548859 P
(43) Date of publication of application: 27.08.2014
(73) Proprietor: Otsuka Pharmaceutical Co., Ltd., Tokyo 101-8535 (JP)
(72) Inventor: OKAMOTO, Ayako, Osaka-shi, Osaka 5400021 (JP)
(74) Representative: Hoffmann Eitle
(86) International application number: PCT/JP2012/077668
(87) International publication number: WO 2013/058411

(56) References cited:
- WO-A1-2009/039324
- WO-A1-2012/026562
- US-A1- 2009 286 805
- US-A1- 2010 179 322

## Description

### TECHNICAL FIELD OF THE INVENTION

The present invention relates to a solution suitable for oral administration of 7-[4-(4-benzo[b]thiophen-4-yl-piperazin-1-yl)butoxy]-1H-quinolin-2-one or a salt thereof.

### BACKGROUND OF THE INVENTION

It is known that 7-[4-(4-benzo[b]thiophen-4-yl-piperazin-1-yl)butoxy]-1H-quinolin-2-one (hereinafter to be referred to as compound (I)) or a salt thereof has dopamine D₂ receptor partial agonist action, serotonin 5-HT_{2A} receptor antagonist action and adrenaline α₁ receptor antagonist action, and further has a serotonin uptake inhibitory action (or serotonin reuptake inhibitory action) in addition to those actions (patent document 1), and has a wide treatment spectrum for central neurological diseases (particularly schizophrenia).

Moreover, compound (I) or a salt thereof is hardly soluble in water and has a bitter taste.

### [Document List]

### [patent document]

patent document 1: JP-A-2006-316052

### SUMMARY OF THE INVENTION

### Problems to be Solved by the Invention

A pharmaceutical solution of compound (I) or a salt thereof, which is effective for oral administration, meets the need specific to patients with central neurological diseases (particularly patients with mental diseases such as schizophrenia and the like) who have difficulty swallowing a solid agent for oral administration. Moreover, a solution for oral administration facilitates handling of doctors to determine dose and the like for patients.

For formulation of a solution for oral administration of compound (I) or a salt thereof, said drug which is poorly soluble in water is desired to be solubilized. In addition, provision of a solution having a less bitter taste, which is easy to take, is desired.

### Means of Solving the Problems

The present inventors have conducted various studies in an attempt to solve the aforementioned problems and found that a solution for oral administration of compound (I) or a salt thereof, wherein the drug is solubilized, can be obtained by adding thereto at least one compound selected from the group consisting of lactic acid, phosphoric acid, glycolic acid, malic acid, tartaric acid, citric acid, succinic acid and acetic acid and adjusting the pH thereof to 2.5 - 4.5. Moreover, they have found that a superior buffering ability can be obtained by adding glycine to the solution. Furthermore, they have found that a solution having a less bitter taste, which is easy to take and affords the above-mentioned effect, can be obtained by adding at least one flavor enhancing and/or masking agent to the solution. The present invention has been completed based on such findings.

Accordingly, the present invention relates to the following.
[1] A solution for oral administration comprising compound (I) or a salt thereof, and at least one compound selected from the group consisting of lactic acid, phosphoric acid, glycolic acid, malic acid, tartaric acid, citric acid, succinic acid and acetic acid, and having pH 2.5 - 4.5.
[2] The solution of the above-mentioned [1], further comprising glycine.
[3] The solution of the above-mentioned [1] or [2], wherein at least one compound selected from the group consisting of lactic acid, phosphoric acid, glycolic acid, malic acid, tartaric acid, citric acid, succinic acid and acetic acid is lactic acid.
[4] The solution of any of the above-mentioned [1] - [3], further comprising at least one flavor enhancing and/or masking agent.
[5] The solution of any of the above-mentioned [1] - [4], further comprising a solubilizing agent.
[6] A solution for oral administration comprising compound (I) or a salt thereof, at least one flavor enhancing and/or masking agent, and at least one compound selected from the group consisting of lactic acid, phosphoric acid, glycolic acid, malic acid, tartaric acid, citric acid, succinic acid and acetic acid, and having pH 2.5 - 4.5.
[7] A solution for oral administration comprising compound (I) or a salt thereof, at least one flavor enhancing and/or masking agent, and at least one compound selected from the group consisting of lactic acid, phosphoric acid, glycolic acid and malic acid, and having pH 2.5 - 4.5.
[8] The solution of the above-mentioned [6] or [7], further comprising a solubilizing agent.
[9] The solution of the above-mentioned [6] or [7], wherein at least one flavor enhancing and/or masking agent is glycine.

Here, the solution for oral administration of the present invention is an aqueous solution.

### Effect of the Invention

According to the present invention, the solubility of compound (I) and a salt thereof can be enhanced, a solution for oral administration containing compound (I) or a salt thereof dissolved in the solution at a desired concentration can be provided. In addition, the solution for oral administration of the present invention containing glycine has superior buffering ability and, even when diluted with drinking water when in use, pH does not vary much, which prevents precipitation of compound (I) or a salt thereof due to pH variation. Furthermore, the solution for oral administration of the present invention containing at least one flavor enhancing and/or masking agent has a suppressed bitter taste and good flavor, and is easy to drink. Description of Embodiments

The solution for oral administration of the present invention contains compound (I) or a salt thereof as an active ingredient. Compound (I) or a salt thereof can be produced by the method described in JP-A-2006-316052, or a method analogous thereto.

The salt of compound (I) usable in the present invention is not particularly limited as long as it is a pharmacologically acceptable salt. For example, inorganic acid salts such as sulfate, nitrate, hydrochloride, phosphate, hydrobromide and the like, organic acid salts such as acetate, sulfonate such as p-toluenesulfonate, methanesulfonate, ethanesulfonate and the like, oxalate, maleate, fumarate,. malate, tartrate, citrate, succinate, benzoate and the like can be mentioned.

The content of compound (I) or a salt thereof in the solution for oral administration of the present invention is generally about 0.01 - about 6 mg/mL, preferably about 0.1 - about 3 mg/mL, more preferably about 0.5 - about 1 mg/mL, as compound (I).

The solution for oral administration of the present invention contains at least one compound selected from the group consisting of lactic acid, phosphoric acid, glycolic acid, malic acid, tartaric acid, citric acid, succinic acid and acetic acid. Of these, lactic acid, phosphoric acid, glycolic acid or malic acid is preferable, lactic acid or phosphoric acid is more preferable, lactic acid is particularly preferable.

Lactic acid may be D-lactic acid, L-lactic acid, a mixture of L-lactic acid and D-lactic acid, or a racemic mixture of L-lactic acid and D-lactic acid.

In the solution for oral administration of the present invention, the content of "at least one compound selected from the group consisting of lactic acid, phosphoric acid, glycolic acid, malic acid, tartaric acid, citric acid, succinic acid and acetic acid" is generally about 0.5 - about 200 mg/mL, preferably about 1 - about 50 mg/mL, more preferably about 5 - about 20 mg/mL.

Since the solution for oral administration of the present invention contains "at least one compound selected from the group consisting of lactic acid, phosphoric acid, glycolic acid, malic acid, tartaric acid, citric acid, succinic acid and acetic acid", the solubility of compound (I) and a salt thereof can be enhanced, and a solution for oral administration containing compound (I) or a salt thereof dissolved in the preparation at a desired concentration can be provided.

The solution for oral administration of the present invention is characterized by pH 2.5 - 4.5.

The pH of the solution for oral administration of the present invention is preferably 2.5 - 4.0, more preferably 3.0 - 3.6, particularly preferably 3.0 - 3.4.

The solution for oral administration of the present invention having pH within the above-mentioned range can enhance the solubility of compound (I) and a salt thereof, and a solution for oral administration containing compound (I) or a salt thereof dissolved in the solution at a desired concentration can be provided.

The solution for oral administration of the present invention preferably has a pH buffered to fall within the above-mentioned range. In the present invention, the method for adjusting pH and the buffering method are not particularly limited, and a method known in the field of pharmaceutical preparation (for example, addition of buffering agent, pH adjuster) can be used.

For example, the pH can be adjusted to the above-mentioned range and buffered by adding an appropriate amount of acid, for example, lactic acid, phosphoric acid, glycolic acid, malic acid, tartaric acid, citric acid, succinic acid or acetic acid, and an appropriate amount of a base, particularly sodium hydroxide, to the solution for oral administration of the present invention. The solution for oral administration of the present invention after buffering can maintain the intended pH range even when diluted with a neutral, slightly-acid or lightly-basic drink when in use.

In the present invention, when lactic acid, phosphoric acid, glycolic acid, malic acid, tartaric acid, citric acid, succinic acid or acetic acid, which is an essential component, is contained in an amount capable of adjusting pH to the above-mentioned range and buffering, a further acid and an appropriate amount of a base may not be contained.

The solution for oral administration of the present invention preferably contains glycine.

In the present invention, addition of glycine can potentiate the buffering ability.

Depending on the preference of patients, the solution for oral administration may be diluted with drinking water such as mineral water, tap water and the like before administration to increase the amount for easy drinking. In the solution for oral administration of the present invention, compound (I) is dissolved in a pH-dependent manner, and therefore, when it is diluted with drinking water, particularly hard water, the pH of the solution for oral administration may change to result in the precipitation of compound (I) or a salt thereof.

The solution for oral administration of the present invention containing glycine has a superior buffering ability, and therefore, even when it is diluted with drinking water, particularly hard water, pH does not change much and is maintained in the above-mentioned range, thus preventing precipitation of compound (I) or a salt thereof.

The content of glycine in the solution for oral administration of the present invention is generally about 0.5 - about 50 mg/mL, preferably about 1 - about 30 mg/mL, more preferably about 5 - about 20 mg/mL.

In the solution for oral administration of the present invention, it is particularly preferable to contain glycine and lactic acid in combination. By the combined addition of glycine and lactic acid, the buffering ability of the solution is enhanced, and even when diluted with drinking water, particularly hard water, pH does not change much and is maintained in the above-mentioned range, thus preventing precipitation of compound (I) or a salt thereof.

When the solution for oral administration of the present invention contains glycine and lactic acid, the weight ratio of glycine and lactic acid (glycine:lactic acid) is generally about 1:0.1 - 10, preferably about 1:0.5 - 5, more preferably about 1:0.5 - 2.

The solution for oral administration of the present invention preferably contains a flavor enhancing and/or masking agent.

As the flavor enhancing and/or masking agent to be used in the present invention, amino acids such as alanine, threonine, proline, serine and the like, natural sweetening agents such as sucrose, fructose, dextrose, maltose, trehalose, glucose, stevia and glycerin and the like, semisynthetic sweetening agents such as lactitol, maltitol, xylitol, sorbitol and mannitol and the like, synthetic sweetening agents such as sucralose, saccharin, acesulfame potassium and aspartame and the like, and flavor such as cherry, orange, peppermint, strawberry, apple, pineapple, anise fruit, peach, raspberry and orange cream and the like can be mentioned. Of these, sucralose and stevia are preferable as sweetening agents. As flavor, an orange flavor is preferable. One or more kinds thereof may be used.

In the solution for oral administration of the present invention, the content of the flavor enhancing and/or masking agent is generally about 0.1 - about 800 mg/mL, preferably about 0.3 - about 100 mg/mL, more preferably about 0.5 - about 20 mg/mL.

Since glycine has sweetness, it also functions as a flavor enhancing and/or masking agent. When glycine is contained in the solution for oral administration of the present invention, the total content of glycine and other flavor enhancing and/or masking agent only needs to be within the above-mentioned range from the aspects of flavor enhancement and/or masking.

The solution for oral administration of the present invention preferably contains a solubilizing agent.

As the solubilizing agent to be used in the present invention, water-miscible solvents such as ethanol, glycerin, propylene glycol, sorbitol, polyethylene glycol (e.g., polyethylene glycol 400), polyvinylpyrrolidone (povidone) and benzylalcohol and the like, a medically acceptable surfactant having a hydrophile-lipophile balance (HLB) of not less than 15 such as fatty acid ester, polyoxyethylene fatty acid ester, polyoxyethylene sorbitan fatty acid ester (e.g., polysorbate 80), polyoxyethylene monoalkyl ether, hydrogenated oil, polyoxyethylene hydrogenated castor oil (e.g., polyoxyethylene hydrogenated castor oil 60) and poloxamer and the like, cyclic oligosaccharides such as α-cyclodextrin, β-cyclodextrin and hydroxypropyl β cyclodextrin (HPβCD) and the like, and the like can be mentioned. Of these, glycerin, propylene glycol, polyethylene glycol (e.g., polyethylene glycol 400), polyoxyethylene sorbitan fatty acid ester (e.g., polysorbate 80) and HPβCD are preferable, and glycerin, propylene glycol and polyethylene glycol (e.g., polyethylene glycol 400) are more preferable. One or more kinds thereof may be used.

In the solution for oral administration of the present invention, the content of the solubilizing agent is generally about 10 - about 500 mg/mL, preferably about 50 - about 400 mg/mL, more preferably about 100 - about 300 mg/mL.

As the solubilizing agent to be used in the present invention, a combination of propylene glycol and glycerin is particularly preferable. The weight ratio of propylene glycol and glycerin (propylene glycol:glycerin) is preferably about 1:0.1 - 10, more preferably about 1:1 - 5, particularly preferably about 1:3.

The solution for oral administration of the present invention preferably contains a stabilizer.

As the stabilizer, a chelating agent such as a sodium salt of edetic acid (edetate disodium (EDTA-2Na), edetate tetrasodium (EDTA-4Na) etc.), tartaric acid, malic acid and citric acid and the like, an antioxidant such as sodium metabisulfite, sodium bisulfite, propyl gallate, sodium ascorbate and ascorbic acid and the like can be mentioned. Of these, EDTA-2Na is preferable. One or more kinds thereof may be used. Since a stabilizer (e.g., sodium salt of edetic acid, particularly EDTA-2Na) is contained, the solution for oral administration of the present invention can achieve long-term preservation stability.

In the solution for oral administration of the present invention, the content of the stabilizer is generally about 0.001 - about 2 mg/mL, preferably about 0.01 - about 1 mg/mL, more preferably about 0.05 - about 0.2 mg/mL.

The solution for oral administration of the present invention preferably further contains a preservative.

As the preservative, benzoic acid, sodium benzoate, methylparaben, ethylparaben, propylparaben, butylparaben, benzyl alcohol, sorbic acid and potassium sorbate, parahydroxybenzoate esters, dehydroacetic acid, sodium dehydroacetate and the like can be mentioned, of which methylparaben and propylparaben are preferable. One or more kinds thereof may be used.

In the solution for oral administration of the present invention, the content of the preservative is generally about 0.1 - about 10 mg/mL, preferably about 0.5 - about 2 mg/mL.

As the preservative to be used in the present invention, a combination of methylparaben and propylparaben is particularly preferable. The weight ratio of methylparaben and propylparaben (methylparaben:propylparaben) is preferably about 1:0.01 - 0.5, more preferably about 1:0.1 - 0.2, particularly preferably about 1:0.15.

The solution for oral administration of the present invention may contain an additive known in the field of pharmaceutical preparation, besides the above-mentioned components.

A preferable example of the solution for oral administration of the present invention is a solution for oral administration containing compound (I) or a salt thereof, and at least one compound selected from the group consisting of lactic acid, phosphoric acid, glycolic acid, malic acid, tartaric acid, citric acid, succinic acid and acetic acid (particularly lactic acid) and having pH 2.5 - 4.5.

In the above-mentioned solution for oral administration, moreover, a solution for oral administration further containing glycine can be mentioned.

In the above-mentioned solution for oral administration, moreover, a solution for oral administration further containing at least one flavor enhancing and/or masking agent (e.g., sucralose, stevia, flavor) can be mentioned.

In the above-mentioned solution for oral administration, moreover, a solution for oral administration further containing a solubilizing agent (e.g., glycerin, propylene glycol, polyethylene glycol, polyoxyethylene sorbitan fatty acid ester, HPβCD, polyoxyethylene hydrogenated castor oil, particularly, a combination of glycerin and propyleneglycol) can be mentioned.

In the above-mentioned solution for oral administration, moreover, a solution for oral administration further containing a preservative (e.g., methylparaben, propylparaben, particularly a combination of methylparaben and propylparaben) and/or a stabilizer (e.g., sodium salt of edetic acid (particularly, EDTA-2Na)) can be mentioned.

The production method of the solution for oral administration of the present invention is not particularly limited, the solution can be produced by mixing the above-mentioned components by a known method, adjusting the pH and, where necessary, filtration.

For example, solution (a) obtained by mixing and dissolving a solubilizing agent (e.g., glycerin, polyethylene glycol, propylene glycol) which is optionally added, at least one compound selected from the group consisting of lactic acid, phosphoric acid, glycolic acid, malic acid, tartaric acid, citric acid, succinic acid and acetic acid, and compound (I) or a salt thereof in water, and solution (b) obtained by mixing and dissolving a solubilizing agent (e.g., glycerin, polyethylene glycol, propylene glycol) which is optionally added, and an additive (e.g., glycine, flavor enhancing and/or masking agent (e.g., sucralose, stevia, flavor), preservative (e.g., methylparaben, propylparaben), stabilizer (e.g., EDTA-2Na)) which is optionally added in water are mixed, pH is adjusted and the mixture is filtered, whereby the solution for oral administration of the present invention can be produced. An additive (e.g., glycine, flavor enhancing and/or masking agent (e.g., sucralose, stevia, flavor), and stabilizer (e.g., EDTA-2Na)) may be added and blended after mixing solutions (a) and (b).

In the above-mentioned step for preparation of solution (a), the order of addition of each component is not particularly limited. For example, at least one compound selected from the group consisting of lactic acid, phosphoric acid, glycolic acid, malic acid, tartaric acid, citric acid, succinic acid and acetic acid is dissolved in a mixture of a solubilizing agent and water, and compound (I) or a salt thereof is added and dissolved in the mixture to give solution (a). Alternatively, compound (I) or a salt thereof is dispersed in a mixture of a solubilizing agent and water, and at least one compound selected from the group consisting of lactic acid, phosphoric acid, glycolic acid, malic acid, tartaric acid, citric acid, succinic acid and acetic acid is added to the obtained dispersion to dissolve the above-mentioned compound (I) or a salt thereof to give solution (a).

In the above-mentioned step for preparation of solution (b), the order of addition of each component is not particularly limited. For example, an additive (e.g., glycine, flavor enhancing and/or masking agent, preservative, stabilizer) is dissolved in a mixture of a solubilizing agent and water to give solution (b). When paraben (e.g., methylparaben, propylparaben) is used as a preservative, a solution (b) may also be obtained by dissolving paraben in a mixture of a solubilizing agent (e.g., propyleneglycol etc.) and water, and a different solution (b) may also be obtained by dissolving a solubilizing agent (e.g., glycerin etc.) and an additive (e.g., glycine, flavor enhancing and/or masking agent, preservative other than paraben, stabilizer) other than paraben in water. These solutions (b) and solution (a) may be directly mixed.

The temperature at which paraben is dissolved in a mixture of a solubilizing agent (e.g., propyleneglycol) and water is generally 45 - 70°C, preferably 50 - 70°C.

A solution for oral administration containing compound (I) or a salt thereof of the present invention can be used for the treatment of schizophrenia and related disorders (e.g., bipolar disorder and dementia) in human patients. The daily dose of the solution for oral administration of the present invention is generally 0.1 - 6 mL (0.05 - 6 mg as compound (I)), preferably 0.5 - 4 mL (0.5 - 4 mg as compound (I)).

The solution for oral administration of the present invention can be directly administered or after dilution. Examples

The present invention is explained in more detail in the following by referring to Examples, which are not to be construed as limitative.

In the Examples, 7-[4-(4-benzo[b]thiophen-4-yl-piperazin-1-yl)butoxy]-1H-quinolin-2-one is described as compound (I).

### Example 1-1

(1) Polyethylene glycol 400 and a part (20 - 30%) of purified water were mixed, and DL-lactic acid was dissolved with stirring. Compound (I) was added to this solution and dissolved by stirring.
(2) Methylparaben and propylparaben were added to a mixture of propylene glycol and a part (10 - 20%) of purified water, they were mixed and dissolved while maintaining the temperature at 45 - 55°C. The container temperature was lowered to 40 - 50°C, edetate disodium, sucralose, stevia and glycine were added, mixed and dissolved, and the solution was cooled to 25 - 30°C with stirring.
(3) The above-mentioned solution (2) was added to the above-mentioned solution (1) with stirring, and they were mixed. A flavor was further added and they were mixed.
(4) 1N Aqueous sodium hydroxide solution was added to the above-mentioned solution (3) to adjust pH to 3.0-3.2, and diluted with purified water to the final concentration. The mixture was filtered with a stainless steel screen to give an aqueous solution for oral administration having the composition of Table 1.

**Table 1**

| component | quantity (mg/mL) |
|---|---|
| compound (I) | 1 |
| polyethylene glycol 400 | 100 |
| propylene glycol | 50 |
| DL-lactic acid | 15.01 |
| methylparaben | 1 |
| propylparaben | 0.2 |
| edetate disodium | 0.1 |
| glycine | 10 |
| sucralose | 0.75 |
| stevia | 0.6 |
| flavor | 0.9 |
| 1N aqueous sodium hydroxide solution | q.s. |
| purified water | q.s. |

| | |
|---|---|
| *: DL-lactic acid used was of content 90.0%, which is about 13.5 mg/mL when converted to DL-lactic acid as content 100%. The same applies to the following Examples and Control Examples. **: Edetate disodium used was dihydrate (C₁₀H₁₄N₂Na₂O₈ 2H₂O). The same applies to the following Examples and Control Examples. | |

### Example 1-2

(1) Polyethylene glycol 400 and a part (20 - 30%) of purified water were mixed, compound (I) was added and dispersed with stirring. DL-lactic acid was added to this solution with stirring to dissolve compound (I).
(2) Methylparaben and propylparaben were added to a mixture of propylene glycol and a part (10 - 20%) of purified water, they were mixed and dissolved while maintaining the temperature at 50 - 70°C. The solution was cooled to 25 - 30°C with stirring.
(3) Edetate disodium, sucralose, stevia and glycine were mixed with a part (10 - 20%) of purified water and dissolved.
(4) The above-mentioned solution (1) and the above-mentioned solution (2) were added to the above-mentioned solution (3) with stirring, and they were mixed. A flavor was further added and they were mixed.
(5) 1N Aqueous sodium hydroxide solution was added to the above-mentioned solution (4) to adjust pH to 3.0-3.2, and diluted with purified water to the final concentration. The mixture was filtered with a stainless steel screen to give an aqueous solution for oral administration having the composition of Table 1.

### Example 2

In the same manner as in Example 1 except that the amount of compound (I) to be added was reduced to half, an aqueous solution of compound (I) (0.5 mg/mL) for oral administration was obtained.

### Example 3-1

(1) Glycerin and a part (20 - 30%) of purified water were mixed, and DL-lactic acid was dissolved with stirring. Compound (I) was added to this solution and dissolved by stirring.
(2) Methylparaben-and propylparaben were added to a mixture of propylene glycol and a part (10 - 20%) of purified water, they were mixed and dissolved while maintaining the temperature at 45 - 55°C. The container temperature was lowered to 40 - 50°C, edetate disodium, sucralose and glycine were added, mixed and dissolved, and the solution was cooled to 25 - 30°C with stirring.
(3) The above-mentioned solution (2) was added to the above-mentioned solution (1) with stirring, and they were mixed. A flavor was further added and they were mixed.
(4) 1N Aqueous sodium hydroxide solution was added to the above-mentioned solution (3) to adjust pH to 3.0-3.2, and diluted with purified water to the final concentration. The mixture was filtered with a stainless steel screen to give an aqueous solution for oral administration having the composition of Table 2.

**Table 2**

| component | quantity (mg/mL) |
|---|---|
| compound (I) | 1 |
| glycerin | 150 |
| propylene glycol | 50 |
| DL-lactic acid | 15.01 |
| methylparaben | 1 |
| propylparaben | 0.15 |
| edetate disodium | 0.1 |
| glycine | 10 |
| sucralose | 0.75 |
| flavor | 0.9 |
| 1N aqueous sodium hydroxide solution | q.s. |
| purified water | q.s. |

### Example 3-2

(1) An about half amount of propylene glycol and a part (20 - 30%) of purified water were mixed, and compound (I) was added and dispersed by stirring. DL-lactic acid was added to the solution with stirring to dissolve compound (I).
(2) Methylparaben and propylparaben were added to a mixture of the rest of propylene glycol and a part (10 - 20%) of purified water, they were mixed and dissolved while maintaining the temperature at 50 - 70°C. The solution was cooled to 25 - 30°C with stirring.
(3) Glycerin, edetate disodium, sucralose and glycine were added to a part (10 - 20%) of purified water, and the mixture was dissolved.
(4) The above-mentioned solution (1) and the above-mentioned solution (2) were added to the above-mentioned solution (3) with stirring, and they were mixed. A flavor was further added and they were mixed.
(5) 1N Aqueous sodium hydroxide solution was added to the above-mentioned solution (4) to adjust pH to 3.0-3.2, and diluted with purified water to the final concentration. The mixture was filtered with a stainless steel screen to give an aqueous solution for oral administration having the composition of Table 2.

### Example 4

In the same manner as in Example 3 except that the amount of compound (I) to be added was reduced to half, an aqueous solution of compound (I) (0.5 mg/mL) for oral administration was obtained.

### Examples 5 - 8

Aqueous solutions of Examples 5 - 8 having the compositions of Tables 3 - 6 for oral administration can be produced by methods analogous to Examples 1 - 4.

**Table 3**

| Example 5 | |
|---|---|
| component | quantity (mg/mL) |
| compound (I) | 1 |
| polysorbate 80 | 50 |
| propylene glycol | 50 |
| DL-lactic acid | 15.01 |
| methylparaben | 1 |
| propylparaben | 0.15 |
| edetate disodium | 0.1 |
| glycine | 10 |
| sucralose | 0.75 |
| stevia | 0.6 |
| flavor | 0.9 |
| 1N aqueous sodium hydroxide solution | q.s. |
| purified water | q.s. |

**Table 4**

| Example 6 | |
|---|---|
| component | quantity (mg/mL) |
| compound (I) | 1 |
| HPβCD | 50 |
| propylene glycol | 50 |
| DL-lactic acid | 15.01 |
| methylparaben | 1 |
| propylparaben | 0.15 |
| edetate disodium | 0.1 |
| glycine | 10 |
| sucralose | 0.75 |
| stevia | 0.6 |
| flavor | 0.9 |
| 1N aqueous sodium hydroxide solution | q.s. |
| purified water | q.s. |

**Table 5**

| Example 7 | |
|---|---|
| component | quantity (mg/mL) |
| compound (I) | 1 |
| polyoxyethylene hydrogenated castor oil 60 | 100 |
| propylene glycol | 50 |
| DL-lactic acid | 15.01 |
| methylparaben | 1 |
| propylparaben | 0.15 |
| edetate disodium | 0.1 |
| glycine | 10 |
| sucralose | 0.75 |
| stevia | 0.6 |
| flavor | 0.9 |
| 1N aqueous sodium hydroxide solution | q.s. |
| purified water | q.s. |

**Table 6**

| Example 8 | |
|---|---|
| component | quantity (mg/mL) |
| compound (I) | 1 |
| glycerin | 150 |
| propylene glycol | 50 |
| DL-lactic acid | 15.01 |
| benzoic acid | 2 |
| edetate disodium | 0.1 |
| glycine | 10 |
| sucrose | 400 |
| fructose | 200 |
| flavor | 0.9 |
| 1N aqueous sodium hydroxide solution | q.s. |
| purified water | q.s. |

### Experimental Example 1

Changes of pH when a solution for oral administration is diluted with drinking water were examined by the following tests.

### <test method>

The solutions of Examples 9 - 12 having the composition of Table 7 were produced by the following method.
(1) Glycerin and a part (20 - 30%) of purified water were mixed, compound (I) was added and dispersed with stirring. DL-lactic acid was added to this solution with stirring to dissolve compound (I).
(2) Methylparaben and propylparaben were added to a mixture of propylene glycol and a part (10 - 20%) of purified water, they were mixed and dissolved while maintaining the temperature at 50 - 70°C. The solution was cooled to 25 - 30°C with stirring.
(3) The above-mentioned solution (2) was added to the above-mentioned solution (1) with stirring, and the rest of the additive and a part of purified water were added thereto, and the mixture was dissolved by stirring.
(4) 1N Aqueous sodium hydroxide solution or phosphoric acid was added as necessary to the above-mentioned solution (3) to adjust pH to 3.0-3.2, and diluted with purified water to the final concentration.

In the same manner as above except that compound (I) was not added, a solution of control having the composition of Table 7 were produced.

The obtained solutions of Control Example and Examples were diluted 50-fold with drinking water (Crystal Geyser (hardness 38 mg/L, soft water, manufactured by Crystal Geyser Water Co.; importer and seller: Otsuka Foods Co., Ltd.), Evian (hardness 304 mg/L, hard water, manufactured by Danone; importer and seller: ITO EN, LTD.), Contrex (hardness 1468 mg/L, hard water, manufactured by Nestle Group; importer and seller: Suntoryfoods Co., Ltd.) and tap water) and Otsuka distilled water (manufactured by Otsuka Pharmaceutical Factory, Inc.) and the pH variation before and after the dilution was measured.

As for dilution, the solutions (4 mL) of Control Example and Examples were accurately measured and placed in 50 mL measuring cylinder with a transfer pipette, and precisely adjusted to 50 mL with each drinking water. The diluted samples were used as pH measurement samples.

The pH of the solutions of Control Example and Examples before dilution, pH of each drinking water and pH of the diluted samples are shown in Table 8.

**Table 7**

| component | function | quantity (mg/mL) | | | | |
|---|---|---|---|---|---|---|
| | | Control Example | Example 9 | Example 10 | Example 11 | Example 12 |
| compound (I) | active ingredient | - | 1 | 1 | 1 | 1 |
| glycerin | solubilizing agent | 150 | 150 | 150 | 150 | 150 |
| propylene glycol | solubilizing agent | 50 | 50 | 50 | 50 | 50 |
| DL-lactic acid | buffering agent | 15.01 | 15.01 | 15.01 | 8.51 | 8.51 |
| methylparaben | preservative | 1 | 1 | 1 | 1 | 1 |
| propylparaben | preservative | 0.15 | 0.15 | 0.15 | 0.15 | 0.15 |
| edetate disodium | stabilizer | 0.1 | 0.1 | 0.1 | 0.1 | 0.1 |
| glycine | buffering agent | 10 | 10 | - | 10 | - |
| sucralose | flavor enhancing and/or masking agent | 0.75 | 0.75 | 0.75 | 0.75 | 0.75 |
| flavor | flavor enhancing and/or masking agent | 0.9 | 0.9 | 0.9 | 0.9 | 0.9 |
| 1N aqueous sodium hydroxide solution | pH adjuster | - | - | q.s. | - | q.s. |
| phosphoric acid | buffering agent | - | - | - | 1.69 * | - |
| purified water | solvent | q.s. | q.s. | q.s. | q.s. | q.s. |

| | | | | | | |
|---|---|---|---|---|---|---|
| In Table, "-" means without addition. *: Phosphoric acid used was of content 85.5%, which is about 1.44 mg/mL when converted to phosphoric acid as content 100%. | | | | | | |

**Table 8**

| pH before and after dilution | | | | | | |
|---|---|---|---|---|---|---|
| | drinking water (dilution solvent) | Crystal Geyser (pH 7.26) | Evian (pH 7.77) | Contrex (pH 7.72) | tap water (pH 7.84) | Otsuka distilled water (pH 7.65) |
| | pH before dilution | pH of diluted sample | | | | |
| Control Example | 3.10 | 3.26 | 3.85 | 3.84 | 3.18 | 3.13 |
| Example 9 | 3.11 | 3.27 | 3.88 | 3.87 | 3.20 | 3.14 |
| Example 10 | 3.06 | 3.38 | 4.19 | 4.19 | 3.27 | 3.19 |
| Example 11 | 3.13 | 3.33 | 4.30 | 4.34 | 3.23 | 3.15 |
| Example 12 | 3.08 | 3.54 | 5.55 | 5.59 | 3.42 | 3.27 |

The pH after dilution with each drinking water was compared between Example 9 having the same lactic acid content and containing glycine and Example 10 having the same lactic acid content and without glycine. As a result, pH variation was milder in Example 9 with any drinking water than in Example 10, thus suggesting an enhanced buffering ability. The pH after dilution with each drinking water was compared between Example 11 having the same lactic acid content and containing glycine and Example 12 having the same lactic acid content and without glycine. As a result, pH variation was milder in Example 11 with any drinking water than in Example 12, thus suggesting an enhanced buffering ability.

The above-mentioned results demonstrate that addition of glycine enhances buffering ability.

### INDUSTRIAL APPLICABILITY

According to the present invention, a solution suitable for oral administration of compound (I) or a salt thereof can be provided.

This application is based on US provisional application No. 61/548,859, the contents of which are incorporated in full herein.

## Claims

1. A solution for oral administration having pH 2.5 - 4.5, which comprises 7-[4-(4-benzo[b]thiophen-4-yl-piperazin-1-yl)butoxy]-1H-quinolin-2-one or a salt thereof, and at least one compound selected from the group consisting of lactic acid, phosphoric acid, glycolic acid, malic acid, tartaric acid, citric acid, succinic acid and acetic acid.

2. The solution according to claim 1, further comprising glycine.

3. The solution according to claim 1 or 2, wherein at least one compound selected from the group consisting of lactic acid, phosphoric acid, glycolic acid, malic acid, tartaric acid, citric acid, succinic acid and acetic acid is lactic acid.

4. The solution according to any of claims 1 to 3, further comprising at least one flavor enhancing and/or masking agent.

5. The solution according to claim 4, wherein the enhancing and/or masking agent is sucralose.

6. The solution according to any of claims 1 to 5, further comprising a solubilizing agent.

7. The solution according to claim 6, wherein the solubilizing agent is propylene glycol and/or glycerin.

8. The solution according to any of claims 1 to 7, further comprising a preservative and a stabilizer.

9. The solution according to any of claims 2 to 8, wherein the content of glycine is 5 - 20 mg/mL

10. The solution according to any of claims 3 to 9, wherein the content of lactic acid is 5 - 20 mg/mL

11. The solution according to any of claims 3 to 9, wherein the weight ratio of glycine:lactic acid is 1:0.5 - 2.

12. The solution according to any of claims 7 to 11, wherein the solubilizing agent is composed of propylene glycol and glycerin at a weight ratio of propylene glycol:glycerin of 1:3.

13. The solution according to any of claims 1 to 12, wherein the pH is 3.0 - 3.4.

14. Composition for oral administration comprising 7-[4-(4-benzo[b]thiophen-4-yl-piperazin-1-yl)butoxy]-1H-quinolin-2-one or a salt thereof, and at least one compound selected from the group consisting of lactic acid, phosphoric acid, glycolic acid, malic acid, tartaric acid, citric acid, succinic acid and acetic acid, and having pH 2.5 - 4.5 for use in a method of treating central neurological diseases.

## Patentansprüche

1. Lösung mit einem pH-Wert von 2,5 bis 4,5 zur oralen Verabreichung, die 7-[4-(4-Benzo[b]thiophen-4-yl-piperazin-1-yl)butoxy]-1H-chinolin-2-on oder ein Salz davon und zumindest eine Verbindung, ausgewählt aus der Gruppe bestehend aus Milchsäure, Phosphorsäure, Glykolsäure, Äpfelsäure, Weinsäure, Zitronensäure, Bernsteinsäure und Essigsäure, umfasst.

2. Lösung gemäss Anspruch 1, die ferner Glycin umfasst.

3. Lösung gemäss Anspruch 1 oder 2, wobei zumindest eine Verbindung, ausgewählt aus der Gruppe bestehend aus Milchsäure, Phosphorsäure, Glykolsäure, Äpfelsäure, Weinsäure, Zitronensäure, Bernsteinsäure und Essigsäure, Milchsäure ist.

4. Lösung gemäss einem der Ansprüche 1 bis 3, die ferner zumindest ein geschmacksverbesserndes und/oder -maskierendes Mittel umfasst.

5. Lösung gemäss Anspruch 4, wobei das verbessernde und/oder maskierende Mittel Sucralose ist.

6. Lösung gemäss einem der Ansprüche 1 bis 5, die ferner ein Lösungshilfsmittel umfasst.

7. Lösung gemäss Anspruch 6, wobei das Lösungshilfsmittel Propylenglykol und/oder Glycerin ist.

8. Lösung gemäss einem der Ansprüche 1 bis 7, die ferner ein Konservierungsmittel und einen Stabilisator umfasst.

9. Lösung gemäss einem der Ansprüche 2 bis 8, wobei der Gehalt an Glycin 5 bis 20 mg/ml beträgt.

10. Lösung gemäss einem der Ansprüche 3 bis 9, wobei der Gehalt an Milchsäure 5 bis 20 mg/ml beträgt.

11. Lösung gemäss einem der Ansprüche 3 bis 9, wobei das Gewichtsverhältnis von Glycin:Milchsäure 1:0,5-2 beträgt.

12. Lösung gemäss einem der Ansprüche 7 bis 11, wobei das Lösungshilfsmittel aus Propylenglykol und Glycerin in einem Gewichtsverhältnis von Propylenglykol:Glycerin von 1:3 zusammengesetzt ist.

13. Lösung gemäss einem der Ansprüche 1 bis 12, wobei der pH-Wert 3,0 bis 3,4 beträgt.

14. Zusammensetzung zur oralen Verabreichung, umfassend 7-[4-(4-Benzo[b]thiophen-4-yl-piperazin-1-yl)butoxy]-1H-chinolin-2-on oder ein Salz davon und zumindest eine Verbindung, ausgewählt aus der Gruppe bestehend aus Milchsäure, Phosphorsäure, Glykolsäure, Äpfelsäure, Weinsäure, Zitronensäure, Bernsteinsäure und Essigsäure, die einen pH-Wert von 2,5 bis 4,5 aufweist, zur Verwendung in einem Verfahren zur Behandlung von Erkrankungen des zentralen Nervensystems.

## Revendications

1. Solution pour administration par voie orale ayant un pH de 2,5 à 4,5, qui comprend de la 7-[4-(4-benzo[b]thiophén-4-yl-pipérazin-1-yl)butoxy]-1H-quinolin-2-one ou un sel de celle-ci, et au moins un composé sélectionné dans le groupe constitué de l'acide lactique, de l'acide phosphorique, de l'acide glycolique, de l'acide malique, de l'acide tartrique, de l'acide citrique, de l'acide succinique et de l'acide acétique.

2. Solution selon la revendication 1, comprenant en outre de la glycine.

3. Solution selon la revendication 1 ou 2, dans laquelle le au moins un composé sélectionné dans le groupe constitué de l'acide lactique, de l'acide phosphorique, de l'acide glycolique, de l'acide malique, de l'acide tartrique, de l'acide citrique, de l'acide succinique et de l'acide acétique est l'acide lactique.

4. Solution selon l'une quelconque des revendications 1 à 3, comprenant en outre au moins un agent renforçant et/ou masquant le gout.

5. Solution selon la revendication 4, dans laquelle l'agent renforçant et/ou masquant est le sucralose.

6. Solution selon l'une quelconque des revendications 1 à 5, comprenant en outre un agent solubilisant.

7. Solution selon la revendication 6, dans laquelle l'agent solubilisant est le propylène glycol et/ou la glycérine.

8. Solution selon l'une quelconque des revendications 1 à 7, comprenant en outre un conservateur et un stabilisant.

9. Solution selon l'une quelconque des revendications 2 à 8, dans laquelle la teneur en glycine est de 5 à 20 mg/ml.

10. Solution selon l'une quelconque des revendications 3 à 9, dans laquelle la teneur en acide lactique est de 5 à 20 mg/ml.

11. Solution selon l'une quelconque des revendications 3 à 9, dans laquelle le rapport en poids glycine:acide lactique est de 1:0,5 à 2.

12. Solution selon l'une quelconque des revendications 7 à 11, dans laquelle l'agent solubilisant est constitué de propylène glycol et de glycérine dans un rapport en poids propylène glycol:glycérine de 1:3.

13. Solution selon l'une quelconque des revendications 1 à 12, dans laquelle le pH est de 3,0 à 3,4.

14. Composition pour administration par voie orale comprenant de la 7-[4-(4-benzo[b]thiophén-4-yl-pipérazin-1-yl)butoxy]-1H-quinolin-2-one ou un sel de celle-ci, et au moins un composé sélectionné dans le groupe constitué de l'acide lactique, de l'acide phosphorique, de l'acide glycolique, de l'acide malique, de l'acide tartrique, de l'acide citrique, de l'acide succinique et de l'acide acétique, et ayant un pH de 2,5 à 4,5, pour utilisation dans une méthode de traitement de maladies neurologiques centrales.
